# EUROPÄISCHE PATENTSCHRIFT

(11) **EP 1 303 255 B1**
(45) Veröffentlichungstag und Bekanntmachung des Hinweises auf die Patenterteilung: **03.05.2006**
(21) Anmeldenummer: 01955357.7
(22) Anmeldetag: 18.07.2001
(51) Int. Cl.: A61K 8/66, A61K 8/97, A61Q 7/02, A61Q 19/00, A61Q 9/04

(54) **VERWENDUNG EINES MITTELS WELCHES DAS HAARWACHSTUM INHIBIERT**
USE OF COMPOSITION WHICH INHIBITS HAIR GROWTH
L'UTILISATION D'UNE COMPOSITION INHIBANT LA CROISSANCE PILEUSE

(30) Priorität: 26.07.2000 FR 0009793
(43) Veröffentlichungstag der Anmeldung: 23.04.2003
(73) Patentinhaber: Cognis France, S.A.S., 31360 Boussens (FR)
(72) Erfinder: ZAMBAUX, Marie-France, F-54510 Tomblaine (FR); HOERNER-WETZEL, Viola, F-54280 Seichamps (FR); GILLON, Véronique, F-54270 Essey les Nancy (FR)
(74) Vertreter: Fabry, Bernd
(86) Internationale Anmeldenummer: PCT/EP2001/008275
(87) Internationale Veröffentlichungsnummer: WO 2002/007697

(56) Entgegenhaltungen:
- WO-A-92/10164
- WO-A-96/28008
- CH-A- 654 327
- DE-U- 20 001 736
- FR-A- 2 703 242

## Beschreibung

### Gebiet der Erfindung

Die Erfindung befindet sich auf dem Gebiet der Kosmetik und/oder Pharmazie und betrifft die Verwendung von Mitteln, enthaltend eine synergistisch wirkende Mischung welche das Haarwachstum inhibiert, vorzugsweise in Deodorantien und/oder Antitranspirantien oder After shave.

### Stand der Technik

Kosmetische Zubereitungen stehen dem Verbraucher heute in einer Vielzahl von Kombinationen zur Verfugung. Dabei wird nicht nur erwartet, dass diese Kosmetika einen bestimmten pflegenden Effekt zeigen oder einen bestimmten Mangel beheben, sondem immer häufiger wird nach Produkten verlangt, die mehrere Eigenschaften gleichzeitig aufweisen und somit ein verbessertes Leistungsspektrum zeigen. Ebenso darf der Anwender erwarten, dass die Zusammensetzung des Produktes eine optimale dermatologische Verträglichkeit besitzt, so dass auch empfindliche Verbraucher nicht mit Irritation reagieren. Darüber hinaus sollten die Mittel jedoch auch weitere Funktionen erfüllen, die zunehmend im Bereich der Pflege und insbesondere der Protektion liegen. Von besonderem Interesse sind Stoffe, die sowohl Wirkstoffe darstellen, die für Haut und Haare gewünschte Eigenschaften vermitteln als auch gleichzeitig die technischen Eigenschaften des kosmetischen Produktes, wie Lagerstabilität, Lichtstabilität und Formulierbarkeit positiv beeinflussen oder zumindest nicht verschlechtern. Hierbei sind zusätzlich eine gute Hautverträglichkeit und besonders der Einsatz natürlicher Produkte beim Kunden gefragt Daneben ist es wünschenswert, durch Kombination bereits bekannter Wirkstoffe, oder durch Auffinden neuer Einsatzgebiete bereits bekannter Substanzklassen deutlich bessere Produkte zu erhalten. Die Kombination bereits bekannter Wirkstoffe führt nicht selten dazu, dass es zu positiv synergistischen Effekten kommt und die Konzentration der einzusetzenden Wirkstoffe vermindert werden kann.

Extrakte von Pflanzen und deren Inhaltstoffe finden immer häufiger Einsatz in der Kosmetik und Pharmakologie. Pflanzenextrakte werden seit vielen Jahren in den unterschiedlichsten Kulturen für medizinische aber auch bereits für kosmetische Zwecke genutzt. Oftmals waren für diese Pflanzenextrakte nur ganz bestimmte einzelne Wirkungen bekannt und das Einsatzgebiet sehr eingeschränkt.

Besonders in den Grenzgebieten zwischen Kosmetik und Pharmazie wächst ein großes Interesse an Pflegestoffen, die pharmazeutische Wirksamkeiten zeigen mit sehr geringen Nebenwirkungen. Werden diese Pflegestoffe in kosmetischen Mitteln angeboten so bietet es dem Verbraucher die Möglichkeit bequem und ohne großen Aufwand Mangelerscheinungen zu beheben oder zu verhindern.

Für spezielle kosmetische Mittel ist es oft wünschenswert, dass diese Mittel neben den hauptsächlich gewünschten Eigenschaften Nebeneffekte zeigen, die gleichzeitig einen weiteren positiven pflegenden Effekt liefem oder unerwünschte Effekte wie beispielsweise starken Haarwuchs der Körperbehaarung inhibieren.

Ein normaler Haarwuchs wird in der Regel toleriert, störend wirkt sich allerdings in vielen Fällen ein übermäßiger Haarwuchs aus. Hier wird versucht, das Haarwachstum zu reduzieren bzw. Körperpartien von Haaren zu befreien. Hierzu sind verschiedene Verfahren bekannt. Zum einen sind mechanische Verfahren zu nennen, die sehr schmerzhaft sein können und zeitaufwendig sind und die Gefahr in sich bergen, dass die entstehenden kleinen Wunden infizieren.

Femer sind Verfahren bekannt, wobei die Haare durch kalten oder heißen Wachs entfernt werden, wobei auch hier die Entfernung nur zeitweise geschieht und vor allem Hautirritationen auftreten können.

Des weiteren gibt es die Rasur, bei der die Haare nur abgeschnitten werden und nach kurzer Zeit - und zumeist in verstärktem Umfang - wieder nachwachsen. Viele Männer und auch Frauen erhalten nach der Rasur Hautirritation besonders bei sensibler Haut. Die herkömmlichen After shave Mittel lindem oftmals diese Hautirritationen und erfrischen die Haut, sie verhindern jedoch nicht, dass die Haare oftmals verstärkt wieder nachwachsen. Besonders Frauen nutzen die Rasur zur Entfernung von Haaren unter anderem in den Achselhöhen.

### Beschreibung der Erfindung

Die Aufgabe der vorliegenden Patentanmeldung hat darin bestanden, Mittel bereitzustellen, die neben pflegenden und schützenden Eigenschaften vor allem den Haarwuchs inhibierende Eigenschaften zeigen.

Eine weitere Aufgabe der vorliegenden Patentanmeldung hat darin bestanden Mittel bereitzustellen, die den Haarwuchs inhibierende Eigenschaften zeigen und dieser Effekt beispielsweise in kosmetischen und/oder pharamzeutischen Mitteln zur Verwendung kommt, die für Körperpartien eingesetzt werden, an denen ein Haarwuchs unerwünscht ist.

Gegenstand der Erfindung ist die Verwendung eines Mittels enthaltend eine synergistisch wirkende Mischung welche das Haarwachstum inhibiert, enthaltend hydrolysierte Proteine aus Soja und mindestens einen Extrakt einer Pflanze die ausgewählt ist aus der Gruppe die gebildet wird von Hypericum perforatum, Hamamelis virginiana, Amica montana und Salix alba.

Eine bevorzugte Ausführungsform der Erfindung ist die Verwendung von oben beschriebenen Mitteln wobei die synergistisch wirkende Mischung weiterhin Substanzen enthält, die ausgewählt sind aus der Gruppe, die gebildet wird von Hamstoff, Menthol, Propylenglycol und Salicylsäure.

Überraschenderweise wurde gefunden, dass durch die Verwendung eines Mittels, welches synergis6sch wirkende Mischungen zur Inhibierung des Haarwachstums enthält, enthaltend hydrolysierte Proteine aus Soja und mindestens einen Extrakt einer Pflanze die ausgewählt ist aus der Gruppe die gebildet wird von Hypericum perforatum, Hamamelis virginiana, Amica montana und Salix alba, sowie insbesondere zusätzlich Hamstoff, Menthol, Propylenglycol und Salicylsäure, die Inhibierung des Haarwachstums erreicht werden kann.

Die synergistische Wirkung auf die Inhibierung des Haarwachstums wird besonders bevorzugt erzielt durch Mischungen, die hydrolysierte Proteine aus Soja, Extrakte von Hypericum perforatum, Extrakte von Hamamelis virginiana, Extrakte von Amica montana und Extrakte von Salix alba und weiterhin Hamstoff, Menthol, Propylenglycol und Salicylsäure enthalten. Eine besonders bevorzugte Ausführungsform der Erfindung ist deshalb die Verwendung von Mitteln enthaltend eine synergistisch wirkende Mischung welche das Haarwachstum inhibiert, enthaltend hydrolysierte Proteine aus Soja, Extrakte von Hypericum perforatum, Extrakte von Hamamelis virginiana, Extrakte von Amica montana und Extrakte von Salix alba und weiterhin Hamstoff, Menthol, Propylenglycol und Salicylsäure,

Unter dem Begriff Pflanze im Sinne der vorliegenden Anmeldung sind sowohl ganze Pflanzen als auch Pflanzenteile (Blätter, Wurzeln, Blüten, Rinde) sowie deren Gemische zu verstehen. Die Extrakte sind je nach gewähltem Ausgangsmaterial und nach gewählter Extraktionsmethode unterschiedlich zusammengesetzt.

Eine bevorzugte Ausführungsform der Erfindung ist die Verwendung von oben beschriebenen Mitteln enthaltend genannte Pflanzenextrakte, wobei die Extrakte aus Hamamelis virginiana bevorzugt aus den Blättern der Pflanze stammen, die Extrakte aus Amica montana bevorzugt aus den Blüten stammen und die Extrakte aus Salix alba bevorzugt aus der Rinde stammen.

Die Verwendung von Mitteln enthaltend die beschriebenen synergistisch wirkenden Mischungen hat vor allem den Vorteil, dass der Haarwuchs am gesunden Haar reduziert wird, d.h. es findet keine schmerzhafte oder aggressive Behandlung der Haut statt, so dass es hier auch nicht zu Wunden oder Hautirritationen kommt. Es wird lediglich der Haarwuchs inhibiert, wobei die Inhibierung bis zum vollständigen Stillstand des Haarwachstums führen kann.

Die synergistisch wirkenden Mischungen haben neben den inhibierenden Eigenschaften auf das Haarwachstum auch astringierende, tonische, beruhigende, erfischende und vwndheilende Eigenschaften.

### Proteine aus Soja

Die Proteine aus Soja werden bevorzugt aus Sojamehl durch Extraktion mir demineralisiertem Wasser gewonnen. Zur Hydrolysierung wird die wäßrige Lösung der Proteine mit Proteasen enzymatisch hydrolysiert. Prinzipiell sind alle Proteasen geeignet, die entweder im basischen oder im alkalischen Milieu hydrolysierend wirken. Die Proteine werden erfindungsgemäß wenigstens einmal im basischen Milieu, bevorzugt bei pH 8,7 und wenigstens einmal im sauren Milieu, bevorzugt bei pH 3,6 hydrolysiert.

Je nach Enzym können sich die pH Werte unterscheiden. Die Reaktionstemperatur für die Hydrolyse liegt zwischen 20 und 80°C. bevorzugt zwischen 30 und 60 °C und insbesondere bei 54 °C.

Unter dem Begriff Pflanze im Sinne der vorliegenden Anmeldung sind sowohl ganze Pflanzen als auch Pflanzenteile (Blätter, Wurzeln, Blüten) sowie deren Gemische zu verstehen.

### Hypericum perforatum

Die Pflanze Hypericum Perforatum L. (Hypericaceae) wird auch als Johanniskraut bezeichnet und gehört zur Familie der Guttiferae. Es handelt sich um eine weit verbreitete krautige Pflanze mit goldgelben rispigen Blütenständen. Die Pflanze enthält bis 1% etherisches Öl mit alpha-Pinen, Monoterpenen und n-Alkanen, außerdem die Flavonoide Quercetin, sein 3-Galactosid (Hyperin) und Rutin sowie Quercetin und Isoquercitrin. Arzneilich genutzt werden das zur Blütezeit gesammelte Kraut als Tee oder in Form daraus gewonnener Tinkturen sowie das aus den frischen Blüten gewonnene tiefrote, klare Öl, das ca. 0,1% Hypericin enthält.

### Hamamelis virginiana

Bei der Pflanze Hamamelis virginiana handelt es sich um einen im Spätherbst gelb blühenden nordamerikanischen Strauch (Hamamefidaceae), der auch als Zaubemuß oder Hexenhaselstaude bezeichnet wird und auch in Europa angepflanzt wird. Blätter und Rinden enthalten Gerbstoffe, die Blätter außerdem Flavon-Glykoside und etherische Öle. Hamamelis-Wasser, ein Destillat aus Hamametis-Bfättem und Zweigen, wirkt wohl in erster Linie durch seinen Gehalt an etherischen Öfen tonisierend auf die Haut; in den stark gefärbten Extrakten sind Gerbstoffe enthalten. Typisch sind die Hamamelistannine, Galloylester, die bei der Hydrolyse Gallussäure und Hamametose (C6H1206, MR 180,16) liefem, einen verzweigtkettigen Zucker. Erfindungsgemäß ist der Einsatz von Extrakten aus den Blättern der Pflanze bevorzugt.

Hamamelis-Präparate finden Verwendung zum Stillen kleinerer Blutungen, gegen Varizen, Hämorrhoiden, Blutergüsse, Krampfadern, Wundsein, Hautjucken, Verbrennungs- und Frostschäden. Bei innerticher Anwendung wirkt Hamamelis-Extrakt, wie andere Gerbstoff-haltige Drogen, gegen Durchfall.

### Amica montana

Es handelt sich um ein Kraut trockener Matten der subalpinen bis alpinen Region mit orangegelbe, schwach würzig riechende, etwas bitter schmeckende Blüten. Amica montana enthält 0,2-0,4% etherisches Öl, Bitterstoffe, besonders Sesquiterpen-Lactone (Helenalin und Derivate), Flavon-glykoside (Astragalin, Isoquercitrin). Erfindungsgemäß ist der Einsatz von Extrakten aus den Blüten der Pflanze bevorzugt

Aus Amica-Blüten oder -Wurzeln gewonnene ölige (Amicaöle), alkoholische Auszüge (Amicatinktur) und Salben mit Amica werden wegen ihrer durchblutungsfördemden Wirkung äußerlich bei Prellungen, Blutergüssen usw. angewandt. Innerlich wirkt Amica günstig bei Magen-Darmstörungen, Mund- und Rachenentzündungen, in konzentrierter Form jedoch haut- und schleimhautreizend.

### Salix Alba

Die Gattung Salix, zweihäusige Bäume oder Sträucher, ist auf der Nordhalbkugel weit verbreitet und wird in etwa 500 Arten eingeteilt. Die Salix Arten werden auch als Weiden bezeichnet und die Rinde dieser Weiden wird von zwei- bis dreijährigen Zweigen geschält und enthält 1-12% Salicin bzw. Salicylalkohol-Derivate, andere phenolische Verbindungen sowie 8-20% Gerbstoffe. Sie stellt sozusagen ein "Prodrug" für Salicylsäure dar und wurde entsprechend gegen Fieber, rheumatischen Beschwerden, Kopfschmerzen und Entzündungen eingesetzt. Wegen der vergleichsweise schlechten Verträglichkeit von Weidenrinde ist sie in der Therapie von Acetylsalicylsäure u. Arylessigsäuren (z. B. Diclofenac, Ibuprofen) abgelöst worden.

Erfindungsgemäß ist der Einsatz von Extrakten aus den Rinden der Pflanze bevorzugt

### Extraktion

Die Herstellung der erfindungsgemäß einzusetzenden Extrakte erfolgt durch übliche Methoden der Extraktion von Pflanzen bzw. Pflanzenteilen. Bezüglich der geeigneten herkömmlichen Extraktionsverfahren wie der Mazeration, der Remazeration, der Digestion, der Bewegungsmazeration, der Wirbelextraktion, Ultraschallextraktion, der Gegenstromextraktion, der Perkolation, der Reperkolation, der Evakolation (Extraktion unter vermindertem Druck), der Diakolation und Festflüssig-Extraktion unter kontinuierlichem Rückfluß, die in einem Soxhlet-Extraktor durchgeführt wird, die dem Fachmann geläufig und im Prinzip alle anwendbar sind, sei beispielhaft auf Hagers Handbuch der Pharmazeutischen Praxis, (5. Auflage, Bd. 2, S.1026-1030, Springer Verlag, Benin-Heideiberg-New-York 1991) verwiesen. Als Ausgangsmaterial können frische oder getrocknete Pflanzen oder Pflanzenteile eingesetzt werden, üblicherweise wird jedoch von Pflanzen und/oder Pflanzenteilen ausgegangen, die vor der Extraktion mechanisch zerkleinert und gegebenenfalls entfettet werden. Hierbei eignen sich alle dem Fachmann bekannten Zerkleinerungsmethoden, als Beispiel sei die Zerkleinerung mit einem Klingen enthaltenen Gerät genannt.

Als Lösungsmittel für die Durchführung der Extraktionen können vorzugsweise Wasser, organische Lösungsmittel oder Gemische aus organischen Lösungsmitteln und Wasser, insbesondere Propylenglycol oder niedermolekulare Alkohole, Ester, Ether, Ketone bzw. halogenhaltige Kohlenwasserstoffe mit mehr oder weniger hohen Wassergehalten (destilliert oder nicht destillert) vorzugsweise wässrig, alkoholische Lösungen mit mehr oder weniger hohen Wassergehalten, verwendet werden. Besonders bevorzugt ist die Extraktion mit destilliertem Wasser, Methanol, Ethanol, Propanol, Butanol und deren Isomere, Aceton, Propylenglycolen, Polyethylenglycolen, Ethylacetat, Dichlormethan, Trichlormethan sowie Mischungen hieraus, insbesondere mit einer Mischung aus destilliertem Wasser und Propylenglycol. Die Extraktion erfolgt in der Regel bei 20 bis 100°C, bevorzugt bei 20 bis 80°C. In einer möglichen Ausführungsform erfolgt die Extraktion unter Inertgasatmosphäre zur Vermeidung der Oxidation der Inhaltsstoffe des Extraktes. Die

Extraktionszeiten werden vom Fachmann in Abhängigkeit vom Ausgangsmaterial, dem Extraktionsverfahren, der Extraktionstemperatur, vom Verhältnis Lösungsmittel zu Rohstoff u.a. eingestellt. Nach der Extraktion können die erhaltenen Rohextrakte gegebenenfalls weiteren üblichen Schritten, wie beispielsweise Aufreinigung, Konzentration und/oder Entfärbung unterzogen werden. Falls wünschenswert, können die so hergestellten Extrakte beispielsweise einer selektiven Abtrennung einzelner unerwünschter Inhaltsstoffe, beispielsweise durch Filtration, unterzogen werden. Die Extraktion kann bis zu jedem gewünschten Extraktionsgrad erfolgen, wird aber gewöhnlich bis zur Erschöpfung durchgeführt. Die vorliegende Erfindung umfasst die Erkenntnis, dass die Extraktionsbedingungen sowie die Ausbeuten der Endextrakte je nach gewünschtem Einsatzgebiet gewählt werden können. Falls gewünscht, können die Extrakte anschließend beispielsweise einer Sprüh- oder Gefriertrocknung unterworfen werden. Die Einsatzmenge der Pflanzenextrakte in den genannten Mitteln richtet sich nach der Konzentration der einzelnen Inhaltstoffe und nach der Art der Anwendungen der Extrakte.

Im Sinne der Erfindung bezeichnet der Begriff Extrakt oder Pflanzenextrakt sowohl getrocknete Extrakte als auch Mischungen von getrockneten Extrakten mit Lösungsmittel, bevorzugt Wasser, insbesondere ein Gemisch aus Wasser und Propylengycol.

Die erfindungsgemäße Verwendung von Mitteln enthaltend die beschriebene synergistisch wirkende Mischung welche das Haarwachstums inhibiert, kann prinzipiell für alle kosmetischen und/oder pharmazeutischen Mittel verwendet werden, die für Körperpartien verwendet werden, an denen ein Haarwuchs unerwünscht ist.

In einer besonderen Ausführungsform der Erfindung werden die Mittel erfindungsgemäß in Deodorantien und/oder Antitranspirantien verwendet. Die in den Deodorantien und/oder Antitranspirantien enthaltende, oben beschriebene synergistisch wirkende Mischung enthaltend hydrolysierte Proteine aus Soja und mindestens einen Extrakt einer Pflanze die ausgewählt ist aus der Gruppe die gebildet wird von Hypericum perforatum, Hamamelis virginiana, Amica montana und Salix alba, sowie insbesondere zusätzlich Hamstoff, Menthol, Propylenglycol und Salicylsäure liefert dabei für Deodorantien und/oder Antitranspirantien neben weiteren Effekten den gewünschten Nebeneffekt einer Inhibierung von unerwünschter Achselhöhlenbehaarung. Bevorzugt ist dabei die Verwendung von Mitteln enthaltend eine synergistisch wirkende Mischung aus hydrolysierten Proteinen aus Soja, Hypericum perforatum Extrakt, Hamamelis virginiana Extrakt, Amica montana Extrakt und Salix alba Extrakt sowie Hamstoff, Menthol, Propylenglycol und Salicylsäure.

### Deodorantien und keimhemmende Mittel

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

### ➢ Keimhemmende Mittel

Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4 dichlorphenyl)hamstoff, 2,4,4' -Trichlor-2' -hydroxy-diphenylether (Triclosan), 4-Chlor-3,5-dimethylphenol, 2,2' -Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-lod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Famesol, Phenoxyethanol, Glycerinmonocaprinat, Gtycerinmonocaprylat, Glycerinmonolaurat (GML), Diglycerinmonocaprtnat (DMC), Salicylsäure-N-alkylamide wie z. B. Salicylsäure-n-octytamid oder Salicylsäure-n-decytamid.

### ➢ Enzyminhibitoren

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Zinkglycinat.

### ➢ Geruchsabsorber

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verteihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschaten, Wurzeln, Hölzern, Kräutern und Gräsern. Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethem zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol. Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, lso-E-Super, Fixolide NP, Evemyl, Iraldein gamma, Phenylessigsäure, Geranylacetat, Benzylacetat, Rosenoxid, Romilat, Irotyl und Floramat allein oder in Mischungen, eingesetzt.

### ➢ Antitranspirantien

Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
➢ adstringierende Wirkstoffe,
➢ Ölkomponenten,
➢ nichtionische Emulgatoren.
➢ Coemulgatoren,
➢ Konsistenzgeber,
➢ Hitfsstoffe wie z. B. Verdicker oder Komplexierungsmittel und/oder
➢ nichtwässrige Lösungsmittel wie z. B. Ethanol, Propylenglykol und/oder Glycerin.

Als adstringierende Antitranspirant-Wirkstoffe eignen sich vor allem Salze des Aluminiums. Zirkoniums oder des Zinks. Solche geeigneten antr'hydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichlorohydrat, Aluminium-Zirko-niumtetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
➢ entzündungshemmende, hautschützende oder wohlriechende ätherische Öle,
➢ synthetische hautschützende Wirkstoffe und/oder
➢ öllösliche Parfümöle.

Übliche wasserlösliche Zusätze sind z.B. Konservierungsmittel, wasserlösliche Duftstoffe, pH-Wert-SteUmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

In einer weiteren Ausführungsform der Erfindung werden die Mittel erfindungsgemäß in After shave verwendet. Die in dem After shave enthaltende, oben beschriebene synergistisch wirkende Mischung enthaltend hydrolysierte Proteine aus Soja und mindestens einen Extrakt einer Pflanze die ausgewählt ist aus der Gruppe die gebildet wird von Hypericum perforatum, Hamamelis virginiana, Amica montana und Salix alba, sowie insbesondere zusätzlich Hamstoff, Menthol, Propylenglycol und Salicylsäure liefert dabei für After shave neben weiteren Effekten den gewünschten Nebeneffekt einer Inhibierung von unerwünschter Behaarung, insbesondere von Bartwuchs. Bevorzugt ist dabei die Verwendung von Mitteln enthaltend eine synergistisch wirkende Mischung aus hydrolysierten Proteinen aus Soja, Hypericum perforatum Extrakt, Hamamelis virginiana Extrakt, Amica montana Extrakt und Salix alba Extrakt sowie Hamstoff, Menthol, Propylenglycol und Salicylsäure.

### After shave

Zu den kosmetischen Mitteln, die im Sinne der Erfindung als After shave bezeichnet werden und die nach der Naß- oder Trockenrasur angewendet werden, zählen prinzipiell alle Mittel, die nach der Rasur angewendet werden, insbesondere Rasierwasser, Rasierlotionen, After-shave-Gele und After-shave-Balsam, Sprays, Schaum, Cremes, Stifte, flüssige und feste Puder. Sie können mit den unterschiedlichsten Duftnoten versehen sein. Bei den nach Naß- u. Trockenrasur verwendbaren After-Shave-Artikeln handelt es sich um Präparate, die wenigstens die Hautreizung nach dem Rasieren mildem, die basischen Rasiermittel neutralisieren, den biologischen Säuremantel der Haut wieder herstellen, erfrischend, kühlend und desinfizierend wirken sollen. Ein After-Shave kann neben den synergistisch wirkenden Mischungen zusammengesetzt sein aus Glycerin (od. Glykol-Derivaten), Citronensäure, Alaun, Desinfiziens, Riechstoffen und Alkohol. Ein aus Sprühdosen versprühbares Produkt erhält man z. B. durch Zugabe von 85%igem Alkohol, Parfüm, Polyvinylpyrrolidon und gasförmigen Treibmittel.

Die mehrfachen erfindungsgemäßen Verwendungsmöglichkeiten von Mitteln enthaltend eine synergistisch wirkende Mischung welche das Haarwachstum inhibiert, enthaltend hydrolysierte Proteine aus Soja und mindestens einen Extrakt einer Pflanze die ausgewählt ist aus der Gruppe die gebildet wird von Hypericum perforatum, Hamamelis virginiana, Amica montana und Salix alba und insbesondere zusätzlich Hamstoff, Menthol, Propylenglycol und Salicylsäure sind für den Markt und für den Verbraucher sehr attraktiv. Die komplexe Aufgabe der Erfindung konnte somit durch den Einsatz dieser Mittel gelöst werden.

Eine bevorzugte Ausführungsform der Erfindung ist die Verwendung von oben beschriebenen Mitteln wobei die in der synergistisch wirkenden Mischung enthaltenden Komponenten in den Mitteln folgende Zusammensetzung haben:
- 0,01 bis 40 Gew.-% hydrolysierte Proteine aus Soja-Extrakt, bevorzugt von 0,01 bis 36,3 Gew.-%, von 0,1 bis 10 Gew.-%, von 0,7 bis 7,3 Gew.-%, besonders bevorzugt von 1,5 bis 3,6 Gew.-% und insbesondere 3,63 Gew.-%
- 0,005 bis 10 Gew.-% Hypericum Perforatum Extrakt bevorzugt von 0,01 bis 7 Gew.-%, von 0,07 bis 3,5 Gew.%, besonders bevorzugt von 0,35 bis 0,7 und insbesondere 0,35 Gew.-% und/oder
- 0,005 bis 10 Gew.-% Hamamelis Vrginiana Extrakt, bevorzugt von 0,01 bis 6 Gew.-%, von 0,06 bis 3 Gew.-%, besonders bevorzugt von 0,3 bis 0,7 und insbesondere 0,3 Gew.-% und/oder
- 0,005 bis 10 Gew.-% Amica Montana Extrakt, bevorzugt von 0,01 bis 6 Gew.-%, von 0,06 bis 3 Gew.-%, besonders bevorzugt von 0,3 bis 0,7 und insbesondere 0,3 Gew.-% und/oder
- 0,001 bis 10 Gew.-% Salix Alba Extrakt, bevorzugt von 0,005 bis 3 Gew.-%, von 0,01 bis 1,5 Gew.-%, besonders bevorzugt von 0,15 bis 0,3 und insbesondere 0,15 Gew.-% und gegebenenfalls
- 0,0005 bis 10 Gew.-% Menthol, bevorzugt von 0,005 bis 1 Gew.%, von 0,01 bis 0,5 Gew.-%, besonders bevorzugt von 0,05 bis 0,1 und insbesondere 0,05 Gew.-% und
- 0.0005 bis 10 Gew.-%.-% Hamstoff, bevorzugt von 0,01 bis 4 Gew.-%, von 0,04 bis 2,0 Gew.-%, besonders bevorzugt von 0,2 bis 0,28 und insbesondere 0,2 Gew.-% und
- 0,05 bis 40 Gew.-% Propylenglycol, bevorzugt von 0,1 bis 35 Gew.-%, von 0,3 bis 25 Gew.-%, besonders bevorzugt von 1 bis 10 und insbesondere 1,5 Gew.-% und
- 0.0005 bis 3 Gew.-% Salicylsäure, bevorzugt von 0,001 bis 0,25 Gew.-%, von 0,0025 bis 0,125 Gew.-%, besonders bevorzugt von 0,0125 bis 0,025 und insbesondere 0,0125 Gew.-%, mit der Maßgabe, dass sich gegebenenfalls die Mengenangaben mit Wasser und/oder weiteren Hilfs- und Zusatzstoffen zu 100 Gew.-% ergänzen.

Die Komponenten können als Trockensubstanz oder in Lösung eingesetzt werden. Bei den hydrolysierten Proteinen aus Soja Extrakt ist der Einsatz von wässrigen Lösungen bevorzugt, insbeondere von wäßrigen Lösungen, die einen Trockensubstanzgehalt von 8 bis 10 Gew.-%, bevorzugt 9 Gew.-% und einen Proteingehalt von 3 bis 5 Gew.%, bevorzugt 4 Gew.-% haben.

Die Extrakte aus Hypericum perforatum, Hamamelis virginiana, Argania montana und Salix alba können als Trockensubstanz oder als Lösung eingesetzt werden. Beide Einsatzmöglichkeiten werden im Sinne der Erfindung als Extrakte bezeichnet. Bevorzugt ist der Einsatz als Lösung. Als Lösungsmittel ist Wasser und/oder ein Wasser-Propylenglycol-Gemisch bevorzugt. Das Verhältnis von Wasser zu Propylenglycol kann zwischen 1:1,5 und 2:1 liegen, bevorzugt liegt es bei 1:1. Der Gehalt an Trockensubstanz dieser einzusetzenden Extrakte liegt zwischen 1 und 5 Gew: %, bevorzugt zwischen 1,5 und 3 Gew.-%, insbesondere bei 2 Gew.-%.

Eine bevorzugte Ausführungsfonn der Erfindung ist die Verwendung von oben beschriebenen Mitteln wobei die synergistisch wirkende Mischung folgende Zusammensetzung hat:
- 72,75 Gew.-% hydrolysierte Proteine aus Soja
- 7 Gew.-% Hypericum Perforatum Extrakt und
- 6 Gew.-% Hamamelis Virginiana Extrakt und
- 6 Gew.-% Amica Montana Extrakt und
- 3 Gew.-% Salix Alba Extrakt und
- 4 Gew.-% Harnstoff und
- 1 Gew.-% Menthol und
- 0,25 Gew.-% Salicylsäure

Der Einsatz von hydrolysierten Proteinen aus Soja entspricht bevorzugt einer wäßrigen Lösung, die einen Proteingehalt von 4 % und einen Trockensubstanzgehalt von 9 % aufweisen. Die Extrakte aus Hypericum perforatum werden bevorzugt als Lösung eingesetzt, wobei das Lösungsmittel aus Wasser und Propylenglycol besteht. Bevorzugt ist ein Gemisch aus jeweils gleichen Teilen Wasser und Propylenglycol. Der Anteil an Trockensubstanz beträgt 2 Gew.-%.

Die Extrakte aus Hamamelis virginiana werden bevorzugt als Lösung eingesetzt, wobei das Lösungsmittel aus Wasser und Propylenglycol besteht Bevorzugt ist ein Gemisch aus jeweils gleichen Teilen Wasser und Propylenglycol. Der Anteil an Trockensubstanz beträgt 2 Gew.-%.

Die Extrakte aus Amica montana werden bevorzugt als Lösung eingesetzt, wobei das Lösungsmittel aus Wasser und Propylenglycol besteht. Bevorzugt ist ein Gemisch aus jeweils gleichen Teilen Wasser und Propylenglycol.

Die Extrakte aus Salix alba werden bevorzugt als Lösung eingesetzt, wobei das Lösungsmittel aus Wasser und Propylenglycol besteht. Bevorzugt ist ein Gemisch aus jeweils gleichen Teilen Wasser und propylenglycol. Der Anteil an Trockensubstanz beträgt 1,5 Gew.-%.

Der Anteil an Propylenglycol in der Mischung liegt bei 25 bis 40 Gew.-%.

Diese Mischung wir in kosmetische Mittel bevorzugt mit 5 Gew.-% bei Temperaturen unter 50 °C eingearbeitet. Diese Msichung ist löslich in Wasser aber unlöslich in Fette und Ölen.

Die erfindungsgemäßen Extrakte enthalten einen Wirkstoffgehalt in den Extrakten von 1 bis 100 Gew.-%, vorzugsweise 10 bis 95 Gew.-%, insbesondere 20 bis 80 Gew.-%. Der Wirkstoffgehalt im Sinne der Erfindung bezeichnet die Summe aller im Extrakt vorhandenen Wirkstoffe bezogen auf das Trockengewicht des Extraktes.

Wirkstoff im Sinne der Erfindung bezieht sich auf die im Extrakt enthaltenen Inhaltstoffe auch wenn deren Gehalt und Identität mit herkömmlichen, dem Fachmann bekannten Methoden noch nicht nachzuweisen sind. Unter Wirkstoffe im Sinne der Erfindung sind weiterhin alle im Extrakt erhaltenen Inhaltsstoffe zu verstehen, deren Wirkung entweder bereits bekannt ist, oder deren Wirkung mit herkömmlichen, dem Fachmann bekannten Methoden noch nicht nachgewiesen werden konnte.

Aktivsubstanz im Sinne der Erfindung bezieht sich auf den Anteil an Substanzen sowie Hilfs- und Zusatzstoffen, die in dem Mittel enthaltend sind, mit Ausnahme des zusätzlich hinzugefügten Wassers.

Der Gesamtanteil an Hilfs- und Zusatzstoffe kann 1 bis 50, vorzugsweise 5 bis 40 Gew.-% - bezogen auf die Endzubereitung der kosmetischen und/oder dermatologischen Zubereitungen - betragen. Die Herstellung der Zubereitungen kann durch übliche Kalt - oder Heißprozesse erfolgen.

Die vorliegenden Erfindung schließt die Erkenntnis ein, dass durch das Zusammenwirken der Inhaltsstoffe der synergistisch wirkenden Mischung, besonders wirkungsvolle kosmetische und/oder pharmazeutische Mittel erhalten werden. Sie zeigen eine hervorragende hautpflegende Wirkung bei gleichzeitig hoher Hautverträglichkeit. Außerdem zeigen sie eine gute Stabilität, insbesondere gegenüber oxidativer Zersetzung der Produkte

Die Begriffe Zubereitungen, Endzubereitungen und Mittel sind im Sinne der Erfindung gleichzusetzen.

Die Mittel enthaltend eine synergistisch wirkende Mischung welche das Haarwachstum inhibiert, können zur Herstellung von kosmetischen und/oder pharmazeutischen Zubereitungen, wie beispielsweise, Sprays, Cremes, Gele, Lotionen, alkoholische und wäßrig/alkoholische Lösungen, Emulsionen, Wachs/ Fett-Massen, Stiftpräparaten, Pudern oder Salben zum Einsatz kommen. Diese Zubereitungen können ferner als weitere Hilfs- und Zusatzstoffe milde Tenside, Ölkörper, Emulgatoren, Perlglanzwachse, Konsistenzgeber, Verdickungsmittel, Überfettungsmittel, Stabilisatoren, Polymere, Siliconverbindungen, Fette, Wachse, UV-Lichtschutzfilter und Antioxidantien, Lecithine, Phospholipide, biogene Wirkstoffe, Filmbildner, Quellmittel, Selbstbräuner, Tyrosinaseinhibitoren (Depigmentierungsmittel), Hydrotrope, Solubilisatoren, Konservierungsmittel, Parfümöle, Farbstoffe und dergleichen enthalten.

### Tenside

Als oberflächenaktive Stoffe können anionische, nichtionische, kationische und/oder amphotere bzw. amphotere Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quatemierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten beispielsweise J.Falbe (ed.), "Surfactants in Consumer Products", Springer Verlag, Berlin,1987, S. 54-124 oder J.Falbe (ed.), "Katalysatoren, Tenside und Mineralöladditive", Thieme Verlag, Stuttgart, 1978, S. 123-217 verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, lsostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Enrcylmyristat, Erucylpalmitat, Erucytstearat, Erucylisostearat, Erucyloleat, Erucylbehenat und Erucylerucat. Daneben eignen sich Ester von linearen C₆-C₂₂₋Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen (vgl. **DE 19756377 A1**), insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈₋Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöffnungsprodukte von epoxidierten Fettsäureestem mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

### Emulgatoren

Als Emulgatoren kommen beispielsweise nichtionogene Tenside aus mindestens einer der folgenden Gruppen in Frage:
➢ Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
➢ Alkyl- und/oder Alkenyloligogfykoside mit 8 bis 22 Kohlenstoffatomen im Alk(en)ylrest und deren ethoxylierte Analoga;
➢ Anlagerungsprodukte von 1 bis 15 Mol Ethylenoxid an Ricinusöl und/oder gehärtetes Ricinusöl;
➢ Anlagerungsprodukte von 15 bis 60 Mol Ethylenoxid an Ricinusöl und/oder gehärtetes Ricinusöl;
➢ Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
➢ Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sor bit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
➢ Mischester aus Pentaerythrit, Fettsäuren, Citronensäure und Fettalkohol gemäß DE 1165574 PS und/oder Mischester von Fettsäuren mit 6 bis 22 Kohlenstoffatomen, Methylglucose und Polyolen, vorzugsweise Glycerin oder Polyglycerin.
➢ Mono-, Di- und Trialkylphosphate sowie Mono-, Di- und/oder Tri-PEG-alkylphosphate und deren Salze;
➢ Wollwachsalkohole;
➢ Polysiloxan-Polyalkyl-Polyether-Copolymere bzw. entsprechende Derivate;
➢ Block-Copolymere z.B. Polyethylenglycol-30 Dipolyhydroxystearate;
➢ Pofymeremulgatoren, z.B. Pemulen-Typen (TR-1,TR-2) von Goodrich;
➢ Polyalkylenglycole sowie
➢ Glycerincarbonat.

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE 2024051 PS** als Rückfettungsmittel für kosmetische Zubereitungen bekannt.

Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäurernonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfetsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

Als Sorbitanester kommen Sorbitanmonoisostearat, Sorbitansesquiisostearat, Sorbitandiisostearat, Sorbitantriisostearat, Sorbitanmonooleat, Sorbitansesquioteat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

Typische Beispiele für geeignete Polyglycerinester sind Polyglyceryl-2 Dipolyhydroxystearate (Dehymuls® PGPH), Polyglycerin-3-Diisostearate (Lameform® TGl), Polyglyceryl-4 lsostearate (Isolan® Gl 34), Polyglyceryl-3 Oleate, Düsostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether(Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Suffonatgruppe tragen. Besonders geeignete zwitterionische Tenside sind die sogenannten Betaine wie die N-Alkyl,N,N-dimethylammoniumglycinate, beispielsweise das Kokosakyldimethylammoniumglycinat, N-Acylaminopropyl-N,N-dimethylammoniumglycinate, beispiels-weise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung Cocamidopropyl Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquatemierte Difettsäuretriethanolaminester-Salze, besonders bevorzugt sind.

### Fette und Wachse

Typische Beispiele für Fette sind Glyceride, d.h. feste oder flüssige pflanzliche oder tierische Produkte, die im wesentlichen aus gemischten Glycerinestern höherer Fettsäuren bestehen, als Wachse kommen u.a. natürliche Wachse, wie z.B. Candelillawachs, Camaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearyfether, Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Konsistenzgeber und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Keselsäuren), Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### Überfettungsmittel

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

### Stabilisatoren

Als Stabilisatoren können Metallsatze von Fettsäuren, wie z.B. Magnesium-, Aluminium- und/oder Zinkstearat bzw. -ricinoleat eingesetzt werden.

### Polymere

Geeignete kationische Polymere sind beispielsweise kationische Cellulosederivate, wie zB. eine quatemierte Hydroxyethylcellulose, die unter der Bezeichnung Polymer JR 400® von Amerchol erhält rich ist, kationische Stärke, Copolymere von Diallylammoniumsalzen und Acrylamiden, quatemierte VinyJpyrrolidonMnyJimidazol-PoJymere, wie z.B. Luviquat® (BASF), Kondensationsprodukte von Polyglycolen und Aminen, quatemierte Kollagenpolypepfide, wie beispielsweise Laurytdimonium Hydroxypropyl Hydrolyzed Collagen (Lamequat®L/Grünau), quatemierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyl-diallylammoniumchlorid (Merquat® 550/Chemviron), Polyaminopolyamide, wie z.B. beschrieben in der FR 2252840 A sowie deren vernetzte wasserlöslichen Polymere, kationische Chitinderivate wie beispielsweise quatemiertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quatemierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/Isobomylacrylat-Copolymere, Methylvinylether/Maleinsäureanhydrid-Copolymere und deren Ester, unvemetzte und mit Polyolen vemetzte Polyacrylsäuren, Acrylamidopropyltrimethylammoniumchlorid/Acrylat-Copolymere, Octylacrylamid/Methylmeth-acrylat/tertButylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage. Weitere geeignete Polymere und Verdickungsmittel sind in Cosm.Toil.108, 95 (1993) aufgeführt.

### Siliconverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. Eine detaillierte Übersicht über geeignete flüchtige Silicone findet sich zudem von Todd et al. in Cosm.Toil. 91, 27 (1976).

### UV-Lichtschutzfilter und Antioxidantien

Unter UV-Lichtschutzfaktoren sind beispielsweise bei Raumtemperatur flüssig oder kristallin vorliegende organische Substanzen (Lichtsdiutzfflter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
➢ 3-Benzylidencampher bzw. 3-Benzylidennorcampher und dessen Derivate, z.B. 3-(4-Methylbenzyliden)campher wie in der EP 0693471 B1 beschrieben;
➢ 4-Aminobenzoesäurederivate, vorzugsweise 4-(Dimethylamino)benzoesäure-2-ethyl-hexylester, 4-(Dimethylamino)benzoesäure-2-octylester und 4-(Dimethylamino)benzoe-säureamylester,
➢ Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
➢ Ester der Salicylsäure, vorzugsweise Salicylsäure-2-ethylhexylester, Salicylsäure-4-iso-propylbenzylester, Salicylsäurehomomenthylester;
➢ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
➢ Ester der Benzalmalonsäure, vorzugsweise 4-Methoxybenzmalonsäuredi-2-ethylhexyl-ester,
➢ Triazinderivate, wie z.B. 2,4,6-Trianilino-(p-carbo-2'-ethyl-1'-hexyloxy)-1,3,5-triazin und Octyl Triazon, wie in der EP 0818450 A1 beschrieben oder Dioctyl Butamido Triazone (Uvasorb® HEB);
➢ Propan-1,3-dione, wie z.B. 1-(4-tert.Butylphenyl)-3-(4'methoxyphenyl)propan-1,3-dion;
➢ Ketotricyclo(5.2.1.0)decan-Derivate, wie in der EP 0694521 B1 beschrieben.

Als wasserlösliche Substanzen kommen in Frage:
➢ 2-Phenylbenzimidazol-5-sulfonsäure und deren Alkali-, Erdalkali-, Ammonium-, Alkylammonium-, Alkanolammonium- und Glucammoniumsalze;
➢ Sulfonsäurederivate von Benzophenonen, vorzugsweise 2-Hydroxy-4-methoxybenzo-phenon-5-sulfonsäure und ihre Salze;
➢ Sulfonsäurederivate des 3-Benzylidencamphers, wie z.B. 4-(2-Oxo-3-bornylidenmethyl)benzolsulfonsäure und 2-Methyl-5-(2-oxo-3-bomyliden)sulfonsäure und deren Salze.

Als typische UV-A-Filter kommen insbesondere Derivate des Benzoylmethans in Frage, wie beispielsweise 1-(4'-ter.Butylphenyl)-3-(4'-methoxyphenyl)propan-1,3-dion, 4-tert.-Buty-4'-methoxydibenzoylmethan (Parsol® 1789), 1-Phenyl-3-(4'-isopropylphenyl)-propan-1,3-dion sowie Enaminverbindungen, wie beschrieben in der **DE 19712033 A1** (BASF). Die UV-A und UV-B-Filter können selbstverständlich auch in Mischungen eingesetzt werden. Besonders günstige Kombinationen bestehen aus den Derivate des Benzoylmethans" z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden deartige Kombinationen mit wasserlöslichen Filtem wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet. Weitere geeignete UV-Lichtschutzfilter sind der Übersicht von P.Finkel in SÖFW-Journal **122**, **543 (1996)** sowie **Parf.Kosm. 3, 11 (1999)** zu entnehmen.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Camosin, D-Camosin, L-Camosin und deren

Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), femer (Metall)-Chetatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glyosylrutin, Ferulasäure, Furfulidenglucitol, Camosin, Butylhydroxytoluol, Butylhydroxyanisol, Nordihydroguajakharzsäure, Nordihydroguajaretsäure, Trihydroxybutyrophenon, Hamsäure und deren Derivate, Mannose und deren Derivate, Superoxid-Dismutase, Zink und dessen Derivate (z.B. Zn0, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### Biogene Wirkstoffe

Unter biogenen Wirkstoffen sind im Rahmen der Erfindung zusätzlich solche zu verstehen, die nicht aus den beschriebenen Pflanzen stammen, wie beispielsweise Tocopherolacetat, Tocopherolpalmitat, Ascorbinsäure, (Desoxy)Ribonucleinsäure und deren Fragmentierungsprodukte, Retinol, Bisabolol, Allantoin, Phytantriol, Panthenol, AHA-Säuren, Ceramide, Pseudoceramide, essentielle Öle, weitere Pflanzenextrakte und zusätzliche Vitaminkomplexe.

### Filmbildner

Gebräuchliche Filmbildner sind beispielsweise Chitosan, mikrokristallines Chitosan, quatemiertes Chitosan, Polyvinylpyrrolidon, Vinylpyrrolidon-Vinylacetat-Copolymerisate, Polymere der Acrylsäurereihe, quatemäre Cellulose-Derivate, Kollagen, Hyaluronsäure bzw. deren Salze und ähnliche Verbindungen.

### Quellmittel

Als Quellmittel für wäßrige Phasen können Montmorilfonite, Clay Mineralstoffe, Pemulen sowie alkylmodifizierte Carbopoltypen (Goodrich) dienen. Weitere geeignete Polymere bzw. Quellmittel können der Übersicht von R.Lochhead in **Cosm.Toit. 108.95 (1993)** entnommen werden.

### Selbstbräuner und Depigmentierungsmittel

Als Selbstbräuner eignet sich Dihydroxyaceton. Als Tyrosinaseinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

### Hydrotrope

Zur Verbesserung des Fließverhaltens können femer Hydrotrope, wie beispielsweise Ethanol, lsopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
➢ Glycerin;
➢ Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
➢ technische Oligoglyceringemische mit einem Eigenkondensationsgrad von 1,5 bis 10 wie etwa technische Diglyceringemische mit einem Diglyceringehalt von 40 bis 50 Gew.-%;
➢ Methylolverbindungen, wie insbesondere Trimethylolethan, Trimethylolpropan, Trimethylolbutan, Pentaerythrit und Dipentaerythrit;
➢ Niedrigalkytgfucoside, insbesondere solche mit 1 bis 8 Kohlenstoffen im Alkylrest, wie beispielsweise Methyl- und Butylglucosid;
➢ Zuckeralkohole mit 5 bis 12 Kohlenstoffatomen, wie beispielsweise Sorbit oder Mannit,
➢ Zucker mit 5 bis 12 Kohlenstoffatomen, wie beispielsweise Glucose oder Saccharose;
➢ Aminozucker, wie beispielsweise Glucamin;
➢ Dialkoholamine, wie Diethanolamin oder 2-Amino-1,3-propandiol.

### Konservierungsmittel

Als Konservierungsmittel eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen.

### Parfümöle

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzem (Pinien-, Sandel-, Guajak-, Zedern-, Rosenholz), Kräutem und Gräsem (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethem zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-lsomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labotanumöl und Lavandinöl. Vorzugsweise werden Bergamotteöl, Dihydromyrcenol, Lilial, Lyral, Cüronellol, Phenylethylalkohol, α-Hexylzimtaldehyd, Geraniol, Benzylaceton, Cyclamenaldehyd, Unalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicyclat, Vertofix Coeur, Iso-E-Super, Fixolide NP, Evemyl, Iraldein gamma, Phenylessigsäure, Geranylacetat, Benzylacetat, Rosenoxid, Romilllat, Irotyl und Floramat allein oder in Mischungen, eingesetzt.

### Farbstoffe

Als Farbstoffe können die für kosmetische Zwecke geeigneten und zugelassenen Substanzen ver wendet werden, wie sie beispielsweise in der Publikation "Kosmetische Färbemittel" der Farbstoffkommission der Deutschen Forschungsgemeinschaft, Verlag Chemie, Weinheim, 1984, S.81-106 zusammengestellt sind. Diese Farbstoffe werden üblicherweise in Konzentrationen von 0,001 bis 0,1 Gew.-%, bezogen auf die gesamte Mischung, eingesetzt.

### Beispiele

### 1. Beispiel: Herstellung des Extraktes der Pflanze Hamamelis

In einer Mischung aus 48 kg destilliertem Wasser und 49 kg Propylenglycol wurden 2 kg getrockneter Hamamelis virginiana Extrakt bei Raumtemperatur unter Rühren gelöst und anschließend filtriert. Der getrocknete Extrakt wurde käuflich erworben.

### 2. Beispiel: Extraktion der Pflanzen Arnica montana

In einer Mischung aus 44 kg destilliertem Wasser und 45 kg Propylenglycol wurden 10 kg Amica montana Blüten bei Raumtemperatur unter Rühren durch Mazeration für 48 Stunden extrahiert. Die Extraktionsmischung wurde anschließend filtriert.

### 3. Beispiel: Herstellung des Extraktes der Pflanze Hypericum perforatum

In einer Mischung aus 48 kg destilliertem Wasser und 49 kg Propylenglycol wurden 2 kg getrockneter Extrakt aus Hypericum perforatum bei Raumtemperatur unter Rühren gelöst. Die Lösung wurde anschließend filtriert. Der getrocknete Extrakt wurde käuflich erworben.

### 4. Beispiel: Herstellung des Extraktes der Pflanze Salix alba

In einer Mischung aus 66 kg destilliertem Wasser und 66 kg Propylenglycol wurden 15 kg Rinde der Weide Salix alba bei 80 °C über 3 Stunden extrahiert. Die Lösung wurde anschließend filtriert

### 5. Herstellung von hydrolysierten Proteinen aus Soja

15 kg Sojamehl wurden bei 55 °C über 4 Stunden mit destilliertem Wasser extrahiert. Die Extraktionsmischung wurde anschließend zentrifugiert. Zur Hydrolyse wurde zunächst bei pH 8,7 und 54 °C über 4 Stunden mit einer Protease enzymatisch hydrolysiert und anschließend bei pH 3,6 und 54 °C über 160 min. mit einer Protease enzymatisch hydrolysiert. Zur Inaktivierung der Enzyme wurde die Mischung für eine Stunde auf 95 °C erhitzt und die erhaltene Proteinmischung filtriert. Die Lösung enthielt 9 Gew.-% Trockensubstanz und 4 Gew.-% Proteine.

### 6. Zusammensetzung der synergistisch wirkenden Mischung

Die synergistisch wirkende Mischung, die den Mitteln für die erfindungsgemäßen Verwendungen beigemischt werden, hat beispielsweise und bevorzugt folgende Zusammensetzung (Angaben in Gew.-%). Diese Mischung ist beispielsweise unter dem Markennamen Pilinhib® VEG erhältlich. Es handelt sich hierbei um eine Marke der COGNIS Gruppe.

| | |
|---|---|
| Hydrolysierte Soja Proteine | 72,75% |
| Hypericum Perforatum Extrakt | 7,00% |
| Hamamelis Virginiana Extrakt | 6.00% |
| Amica Montana Blütenextrakt | 6,00% |
| Harnstoff | 4,00% |
| Salix Alba Rindenextrakt | 3,00% |
| Menthol | 1,00% |
| Salicylsäure | 0,25% |

Diese Mischung enthält unter anderem aus den Extrakten von Hypericum perforatum, Hamamelis virginiana, Amica montana und Salix alba zwischen 25 und 40Gew.-% Propylenglycol aus dem Lösungsmittel Wasser und Propylenglycol.

Der Einfachheit halber wird in den folgenden Beispielen von Pilinhib® VEG gesprochen, gemeint sind jedoch Mischungen, die eine oben erwähnte Zusammensetzung besitzen.

### 7. Herstellung der synergistisch wirkenden Mischung

Das Menthol und die Salicylsäure wurden bei 50 °C in Propylenglycol gelöst. Alle weiteren Komponenten wurden bei Raumtemperatur unter Rühren zugegeben. Die Mischung wurde anschließend zentrifugiert und filtriert.

### 8. Test zur Inhibierung des Haarwachstums

Ein Deodorant als Lotion enthaltend 5 Gew.-% Pilinhib® VEG wurde gegen ein Placebo (Deodorant ohne Pilinhib® VEG) an Achselhöhlen von 12 freiwilligen, gesunden Männern und Frauen im Alter von 18 bis 50 Jahren getestet Bei allen Testpersonen lag natürlicherweise ein starkes und schnelles Haarwachstum in der Achselhöhle vor.

Vor der eigentlichen Behandlung wurde jeweils bestimmt, welches Haarwachstum unter den Achselhöhlen nach 10 Tagen zu erwarten ist. Zu diesem Zweck wurden die Achselhöhlen der Testpersonen rasiert und nach zehn Tagen wurde die Haarlänge und der Haardurchmesser durch Macrophotography bestimmt.

Zur Bestimmung der das Haarwachstum inhibierenden Eigenschaften wurden die Produkte über eine Dauer von zehn Tagen täglich zweimal (morgens und abends) aufgetragen. Vor der Behandlung wurden beide Achselhöhlen rasiert. Zum direkten Vergleich wurde jeweils eine Achselhöhle mit der Lotion enthaltend 5 Gew.-% Pilinhib® VEG und die andere Achselhöhle mit der Lotion ohne Pilinhib® VEG behandelt. Nach der zehntägigen Behandlung wurde die Haarlänge und der Haardurchmesser durch Macrophotography bestimmt.

**Tabelle 1: Bestimmung der Haarlänge und des Haarclurchmessers**

| | D20/D10 |
|---|---|
| Deodorant mit 5 Gew.-% Pilinhib® VEG | 42 % |
| Placebo Deodorant | 0 % |

| | |
|---|---|
| D10 =10 Tage nach der Rasur ohne Behandlung D20 =10 Tage nach der Rasur mit Behandlung | |

Nach nur 10 Tagen Behandlung war bei den Freiwilligen die Haarlänge und der Haardurchmesser im Vergleich zur fehlenden Behandlung im Durchschnitt um 42 % veningert. Bei der Behandlung mit Placebo konnte keine Veränderung an der Haarlänge im Vergleich zur fehlenden Behandlung festgestellt werden.

## Patentansprüche

1. Verwendung eines Mittels enthaltend eine synergistisch wirkende Mischung welche das Haarwachstum inhibiert, enthaltend hydrolysierte Proteine aus Soja und mindestens einen Extrakt einer Pflanze die ausgewählt ist aus der Gruppe die gebildet wird von Hypericum perforatum, Hamamelis virginiana, Amica montana und Salix alba

2. Verwendung nach Anspruch 1, **dadurch gekennzeichnet, dass** die synergistisch wirkende Mischung weiterhin Substanzen enthält, die ausgewählt sind aus der Gruppe, die gebildet wird von Hamstoff, Menthol, Propylenglycol und Saücylsäure.

3. Verwendung nach einem der Ansprüche 1 und/oder 2, **dadurch gekennzeichnet, dass** die Extrakte aus Hamamelis virginiana bevorzugt aus den Blättern der Pflanze stammen, die Extrakte aus Amica montana bevorzugt aus den Blüten stammen und die Extrakte aus Salix alba bevorzugt aus der Rinde stammen.

4. Verwendung eines Mittels gemäß einem der Ansprüche 1 bis 3 als Deodorant und/oder Antitranspirant.

5. Verwendung eines Mittels gemäß einem der Ansprüche 1 bis 3 als After shave.

6. Verwendung nach einem der Ansprüche 1 bis 5, **dadurch gekennzeichnet, dass** die in der synergistisch wirkenden Mischung enthaltenden Komponenten in den Mitteln gemäß Ansprüche 1 bis 5 folgende Zusammensetzung haben:
- 0,01 bis 40 Gew.-% hydrolysierte Proteine aus Soja-Extrakt
- 0,005 bis 10 Gew.-% Hypericum perforatum Extrakt und/oder
- 0,005 bis 10 Gew.-% Hamamelis virginiana Extrakt und/oder
- 0,005 bis 10 Gew.-% Amica montana Extrakt und/oder
- 0,001 bis 10 Gew.-% Salix alba Extrakt und gegebenenfalls
- 0,0005 bis 10 Gew.-% Menthol
- 0,0005 bis 10 Gew.-%-% Harnstoff
- 0,005 bis 40 Gew.-% Propylenglycol
- 0,0005 bis 3 Gew.-% Salicylsäure
mit der Maßgabe, dass sich gegebenenfalls die Mengenangaben mit Wasser und/oder weiteren Hilfs- und Zusatzstoffen zu 100 Gew.-% ergänzen.

7. Verwendung nach einem der Ansprüche 1 bis 5, **dadurch gekennzeichnet, dass** die synergistisch wirkende Mischung folgende Zusammensetzung hat
• 72,75 Gew.-% hydrolysierte Proteine aus Soja
• 7 Gew.-% Hypericum perforatum Extrakt und
• 6 Gew.-% Hamamelis virginiana Extrakt und
• 6 Gew.-% Amica montana Extrakt und
• 3 Gew.-% Salix alba Extrakt und
• 4 Gew.-% Harnstoff und
• 1 Gew.-% Menthol und
• 0,25 Gew.-% Salicylsäure

## Revendications

1. Utilisation d'un agent contenant un mélange agissant de façon synergique qui inhibe la repousse du poil, contenant des protéines de soja hydrolysées et au moins un extrait d'une plante qui est sélectionnée dans le groupe formé d'Hypericum Perforatum, d'Hamamelis Virginiana, d'Arnica Montana et de Salix Alba.

2. Utilisation selon la revendication 1, **caractérisée en ce que** le mélange agissant de façon synergique contient de plus des substances qui sont sélectionnées dans le groupe qui est formé de l'urée, du menthol, du propylène glycol et de l'acide salicylique.

3. Utilisation selon l'une des revendications 1 et/ou 2, **caractérisée en ce que** les extraits d'Hamamelis Virginiana proviennent de préférence des feuilles de la plante, que les extraits d'Arnica Montana proviennent de préférence des fleurs et que les extraits de Salix Alba proviennent de préférence de l'écorce.

4. Utilisation d'un agent selon l'une des revendications 1 à 3 en tant que déodorant et/ou anti-transpirant.

5. Utilisation d'un agent selon l'une des revendications 1 à 3 en tant qu'après-rasage.

6. Utilisation selon fune des revendications 1 à 5, **caractérisée en ce que** les composants des agents selon les revendications 1 à 5 contenus dans le mélange agissant de façon synergique ont la composition suivante :
- 0,01 à 40 % en masse de protéines hydrolysées issues d'extrait de soja,
- 0,005 à 10 % en masse d'extrait d'Hypericum Perforatum, et/ou
- 0,005 à 10 % en masse d'extrait d'Hamamelis Virginiana, et/ou
- 0,005 à 10 % en masse d'extrait d'Arnica Montana, et/ou
- 0,001 à 10 % en masse d'extrait de Salix Alba et, le cas échéant,
- 0,0005 à 10 % en masse de menthol,
- 0,0005 à 10 % en masse d'urée,
- 0,005 à 40 % en masse de propylène glycol,
- 0,0005 à 3 % en masse d'acide salicylique,
sous réserve que les indications de quantités soient complétées à 100 % , le cas échéant, avec de l'eau et/ou d'autres auxiliaires et additifs.

7. Utilisation selon l'une des revendications 1 à 5, **caractérisée en ce que** le mélange agissant de façon synergique présente la composition suivante :
• 72,75 % en masse de protéines de soja hydrolysées,
• 7 % en masse d'extrait d'Hypericum Perforatum, et
• 6 % en masse d'extrait d'Hamamelis Virginiana, et
• 6 % en masse d'extrait d'Arnica Montana, et
• 3 % en masse d'extrait de Salix Alba, et
• 4 % en masse d'urée, et
• 1 % en masse de menthol, et
• 0,25 % en masse d'acide salicylique.

## Claims

1. The use of a composition containing a synergistically active mixture which inhibits hair growth and which contains hydrolyzed soya proteins and at least one extract of a plant selected from the group consisting of Hypericum perforatum, Hamamelis virginiana, Arnica montana and Salix alba.

2. The use claimed in claim 1, **characterized in that** the synergistic mixture additionally contains substances selected from the group consisting of urea, menthol, propylene glycol and salicylic acid.

3. The use claimed in claim 1 and/or 2, **characterized in that** the Hamamelis virginiana extracts preferably emanate from the leaves of the plant, the Arnica montana extracts preferably emanate from the flowers and the Salix alba extracts preferably emanate from the bark.

4. The use of the composition claimed in any of claims 1 to 3 as a deodorant and/or antiperspirant.

5. The use of the composition claimed in any of claims 1 to 3 as an aftershave.

6. The use claimed in any of claims 1 to 5, **characterized in that** the components present in the synergistic mixture in the compositions claimed in claims 1 to 5 have the following composition:
- 0.01 to 40% by weight hydrolyzed proteins from soya extract
- 0.005 to 10% by weight Hypericum perforatum extract and/or
- 0.005 to 10% by weight Hamamelis virginiana extract and/or
- 0.005 to 10% by weight Arnica montana extract and/or
- 0.001 to 10% by weight Salix alba extract and optionally
- 0.0005 to 10°/ by weight menthol
- 0.0005 to 10% by weight urea
- 0.005 to 40% by weight propylene glycol
- 0.0005 to 3% by weight salicylic acid
with the proviso that the quantities shown add up to 100% by weight optionally with water and/or other auxiliaries and additives.

7. The use claimed in any of claims 1 to 5, **characterized in that** the synergistic mixture has the following composition:
• 72.75% by weight hydrolyzed soya proteins
• 7% by weight Hypericum perforatum extract
• 6% by weight Hamamelis virginiana extract and
• 6% by weight Arnica montana extract and
• 3% by weight Salix alba extract and
• 4% by weight urea and
• 1% by weight menthol and
• 0.25% by weight salicylic acid.
